# EUROPEAN PATENT SPECIFICATION

(11) **EP 3 247 432 B1**
(45) Date of publication and mention of the grant of the patent: **25.12.2019**
(21) Application number: 16709815.1
(22) Date of filing: 25.01.2016
(51) Int. Cl.: A61M 5/32

(54) **SAFE NEEDLE ASSEMBLY FOR PREVENTING NEEDLE PRICKS**
NADELSICHERHEITSANORDNUNG ZUR VERMEIDUNG VON NADELSTICHEN
DISPOSITIF DE SECURITE D'AIGUILLE POUR LA PREVENTION DE LA PIQURE

(30) Priority: 23.01.2015 IT ME20150001
(43) Date of publication of application: 29.11.2017
(73) Proprietor: LA RG S.N.C. Di Rao Genovese Francesco, Cap 98051 Barcellona Pozzo di Gotto (ME) (IT)
(72) Inventor: RAO GENOVESE, Francesco, 98051 Barcellona Pozzo di Gotto (ME) (IT)
(74) Representative: Maiello, Helenio Francesco
(86) International application number: PCT/IB2016/050359
(87) International publication number: WO 2016/116911

(56) References cited:
- EP-A1- 2 298 397
- US-A- 4 892 525
- US-A- 5 163 915
- US-A1- 2004 111 067

## Description

### Technical Field

The present invention finds application into the field of the medical devices for generic uses and it has particularly as object a safe needle assembly for preventing needle pricks adapted to be applied to syringes or similar injection apparatuses.

### State fo the art

As known, the main risk for a health operator using a needle is represented by the eventuality of being pricked during the recapping of the needle and to the disposal thereof after use.

The injection devices, such as syringes and in general all the apparatuses operating with an interchangeable needle, require the presence of a needle guard which protects the needle prior to use same and that by law cannot be repositioned on the needle at the end of the operation.

As matter of fact, the common needle guard would not allow to recap the needle in a safe manner because they have an inlet with a diameter too close to that of the needle, so that the risk for the operator to insert the needle in a not correct manner would be too high, with consequent prick.

Secondarily, the common needle guard are sized to house solely metal part of the needle, keeping at the outside the end cylindrical hub that allows it to be anchored to the specific device.

For these reasons, the legislation relative to prevention from sharp or pricks injuries in the hospital and healthcare sector, in particular EU Directive No. 2010/32 provides for the absolute prohibition of the practice of manual recapping in the absence of protective and safety devices against pricks and the adoption of medical devices equipped with protection and security mechanisms.

The same law also calls for definition and implementation of procedures for safe use and disposal of sharped medical devices and of waste contaminated with blood and dangerous biological materials.

A protection device from needle pricks is known from WO2011117840 which describes a device consisting of a disk-shaped cap provided with a central through hole designed to house and retain a needle provided with a needle guard to facilitate the removal and disposal of the syringe in a safe condition for the health care worker.

However, such devices do not meet the requirements of the standards currently in force which require that the needle guard cannot be repositioned after its removal, since it does not constitute a safety device.

Other safety systems include a side flap joined to the syringe in non-removable manner which intervenes to cover the needle after removing the needle guard and having executed the injection.

Such solutions are, however, impractical and uncomfortable due to the constant presence of the fin during the entire operation.

Moreover, in these cases it will be necessary to dispose of the syringe, usually in plastic material, together with the needle, with the result that the needle metallic material will affect the recyclability of the plastic material of the syringe.

A further safety device is disclosed, for example, in US4781697, which, however, still provides for the re-use of the needle guard contrary to the regulations in force.

Other solutions of safety devices are known from EP2298397, US5163915 and US4892525. These solutions, in addition to not guaranteeing maximum safety for the operator at all stages of recapping, have the drawback of not ensuring that the needle will be separated from the syringe together with the safety device to allow the unitary disposal.

Furthermore, during recapping the devices disclosed in US5163915 and US4892525 require that the operator handles the needle guard while maintaining the fingers forward the needle, even if shielded from a funnel-sized body.

The device of EP2298397 also do not allow to perform insertion of the needle on the syringe, but only its removal.

Not least, such devices may still make possible the reopening of the needle guard with potential access to the syringe, resulting not suitable for the actual regulations.

### Scope of the invention

The object of the present invention is to overcome the above mentioned drawbacks, providing a safe needle assembly for the prevention against needle pricks that is particularly effective and simple to use and which fully meets all regulations concerning the prevention of injuries from cutting or pricks in the hospital and healthcare sector.

Still another object is to provide a safe needle assembly for the prevention against needle pricks that simplify and make safe the operation of needle recapping and subsequent steps of removal and disposal of the used needle.

A particular object is to provide a safe needle assembly for the prevention against needle pricks which allows to remove and dispose of the needle in a safe manner and without the possibility for the health care worker to come into contact with the needle. Still another object is to provide a safe needle assembly for the prevention against needle pricks which is simple and safe to place on the needle to be protected.

Still another object is to provide a safe needle assembly for the prevention against needle pricks that allows to cover the entire needle, including its hub, to transform a common syringe or similar injection device in a "Safety Syringe".

Still another object is to provide a safe needle assembly for the prevention against needle pricks that allows the safe insertion of the needle on the syringe or injection device.

Not least object is to provide a safe device that is usable in an effective and safe manner even with special type of needles, in particular with carpule for dental use.

These objects, as well others which will appear more clearly hereinafter, are achieved by a safe needle assembly for the prevention against needle pricks according to claim 1.

Thanks to this combination of features, subsequent to its use the needle may be recapped safely without having to reinsert the needle guard previously removed and avoiding that in the recapping and disposing of the operator may come into contact with the pointed end thereof.

Furthermore, making the needle integral in rotation to the cap will prevent that the needle can be separated from the cap during or after the removal or that its removal might fail forcing the operator to perform repeated attempts that would force him to operate directly on the hub.

In the present text the words "proximal" and "distal" indicate, in the usual manner, the ends of the tubular body which in use are closest and farthest, respectively, compared to the operator's grip.

The assembly will also help to remove the needle from the syringe or other injection device in a simple and safe manner and to subsequently dispose of it in the appropriate containers.

Advantageous embodiments of the invention are obtained in accordance with the dependent claims.

### Brief disclosure of the drawings

Further features and advantages of the invention will become more apparent from the detailed description of some preferred but not exclusive embodiments of a safe needle assembly according to the invention, shown by way of non-limiting example with the aid of the accompanying drawing tables wherein:
**FIG. 1** is a front view of a safe needle assembly in a first embodiment assembled according to a first operating mode;
**FIG. 2** is a front view of the safe needle assembly of Fig. 1 in a second operating mode;
**FIG. 3** is an exploded front view of the safe needle assembly of Fig. 1;
**FIG. 4** is a front axially sectional view of the assembly of Fig. 1;
**FIG. 5** is a front axially sectional view of the assembly of Fig. 2;
**FIG. 6** is an enlarged view of a detail of the closing cap of the assembly of Fig.1;
**FIG. 7** is a front axially sectional view of an assembly in a second preferred embodiment;
**FIG. 8** shows three cross sectional views of the assembly of Fig. 1 according A-A, B-B, C-C plans;
**FIG. 9** is a front view of a safe needle assembly in a third preferred embodiment assembled according to the first operating mode;
**FIG. 10** is an exploded front view of the assembly of Fig. 9;
**FIG. 11** is a front view of the assembly of Fig. 9 in a third operative mode;
**FIG. 12** is a cross sectional view of the assembly of Fig. 9;
**FIG. 13** is an exploded front view of a safe needle assembly in a fourth preferred embodiment;
**FIG. 14** is a cross sectional view of the assembly of Fig. 13 in assembled condition according the first operating mode;
**FIG. 15** is a front view of a safe needle assembly in a fifth preferred embodiment assembled according to the first operating mode.

### Best modes of carrying out the invention

The following figures show some preferred but non-limiting embodiment of a safe needle assembly for protection against needle pricks according to the invention designed to be used in a known manner in combination with a needle applied to a syringe **S** or other injection apparatus, not illustrated as of known type.

In general, the needle will be provided with a support tubular hub, commonly also called barrel, with which the needle may be fixed in a removable manner to the syringe **S** or similar apparatus.

The assembly may be designed to be inserted on the needle after the insertion thereof on the syringe to be removed from the syringe and disposed of in a safe condition, thus avoiding its recapping through the first needle guard, in accordance with current regulations.

Alternatively, the assembly may also be used for the safe insertion of the needle on the syringe **S** or other safety device, ensuring maximum protection for the operator at all stages of needle use.

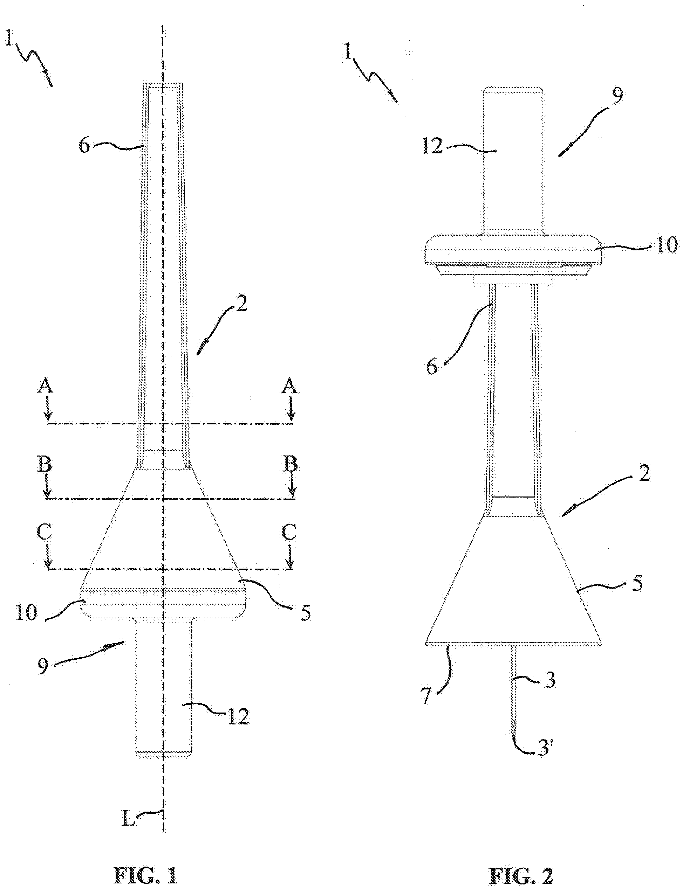

**Fig. 1** shops an assembly, generally indicated by **1,** which comprises a protective cap **2** for housing the needle **3** in a safe condition for the health operator.

The cap **2** has a tubular cavity **4** with an open proximal end portion **5** to allow insertion of the needle **3** and a closed distal end portion **6.**

The proximal end portion **5** has frustoconical shape tapered towards the distal portion **6** with an enlarged inlet **7** to facilitate the insertion of the hub **8** supporting the needle inside the hollow protective cap **2** and to guide the insertion of the needle **3** inside the distal portion **6.**

The assembly **1** also comprises a closure cap **9** having a disk-shaped element **10** which can be removably coupled in a snugly fit manner to the tapered proximal end portion **5** for closing the enlarged inlet **7.**

The discoidal element **10** also comprises a central through hole **11** for positioning the discoidal element **10** on the distal end portion **6** when the same is not coupled to the proximal end portion **5** of the protective cap **2,** as shown in **Fig. 2****.**

In this way, the closure cap **9** held dual function, as in the moment in which it is applied to the distal end portion **6** of the protection element **2** will allow to remove in a simple and safe manner the assembly consisting of the protection element **2,** needle **3** and its hub or barrel **8,** while allow to cover the entire needle **3** when it is applied to the proximal end portion **5,** for the disposal of the needle **3** in a safe condition.

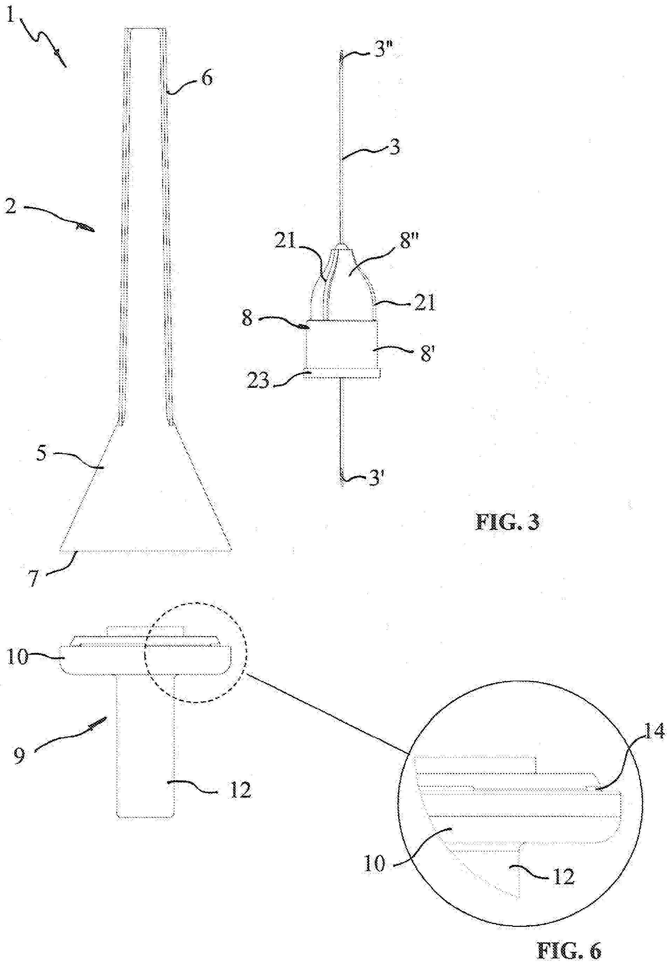

This configuration would be particularly advantageous in the case in which the assembly **1** is to be used with needles of the carpule type for dental use in which there is a needle **3** having both ends pointed and protruding from opposite sides of the support hub **8,** as visible in **Fig. 3****.**

In this figure it could be observed that a first end **3'** of the needle **3** is designed to draw a fluid to be administered while a second end **3"** is designed to supply the fluid to the patient.

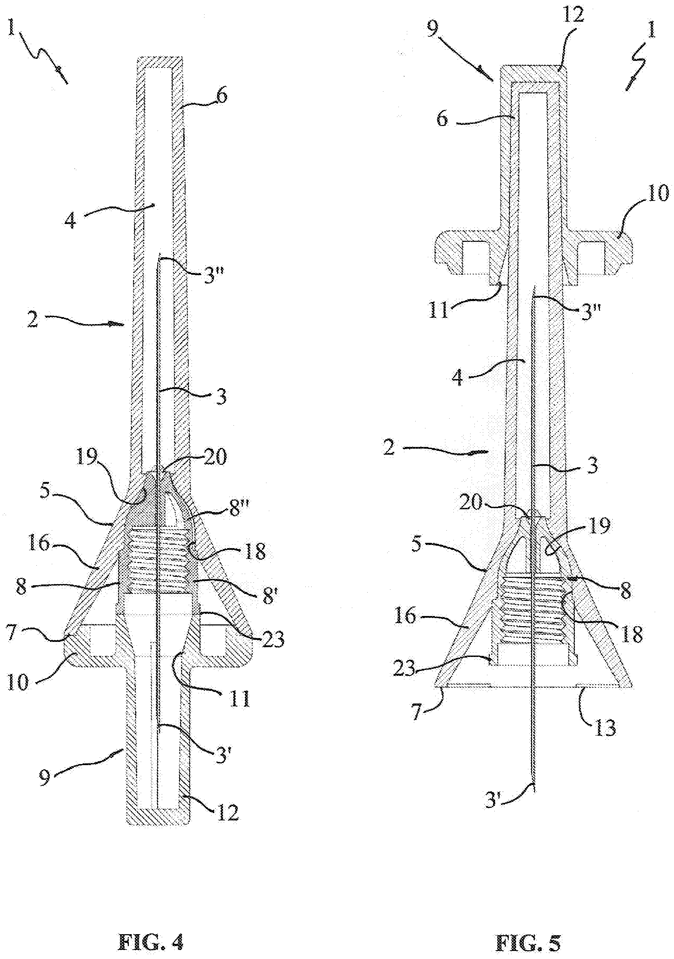

From the section of **Fig. 4** it could be also observed that the discoidal element **10** has a maximum outside diameter substantially corresponding to the outer diameter of the enlarged inlet **7** with a central hole **11** substantially coaxial, in the assembled condition, with the longitudinal axis **L** of the protective cap **2.**

The closure cap **9** comprises a tubular body **12** which extends from the discoidal element **10** coaxially with the through hole **11** and is internally hollow and closed at the end opposite to the hole **11** for housing in a safe manner the first end **3'** of the needle **3.**

From the section of **Fig. 5** it could be observed that the tubular body **12** has an inner shape complementary to the distal end portion **6** of the protection element **2** for snugly fit it.

The firm but removable coupling between the closure cap **9** and the proximal end portion **5** of the protective element **2** is realized by mutual coupling means associated with respective peripheral edges of the discoidal element **10** and the proximal end portion **5** of the cap **2.**

The mutual coupling means will have form complementary with each other and may be selected from the screw and nut means, snap coupling, bayonet, male and female means and the like.

For example, in the configurations described above the protection cap **2** has a proximal end portion **5** provided with one or more fins **13** protruding radially and inwardly from the inner edge of the inlet **7** to be inserted into corresponding first peripheral radial grooves **14** of the discoidal element **10** upon the relative rotation between the closure cap **9** and the protective cap **2,** so as to realize the mutual stable coupling between the parts.

**Fig. 6** shows in an enlarged view a detail of the closure cap **9** which shows one of the first peripheral grooves **14** for the snap closure of the tubular protective element **2.**

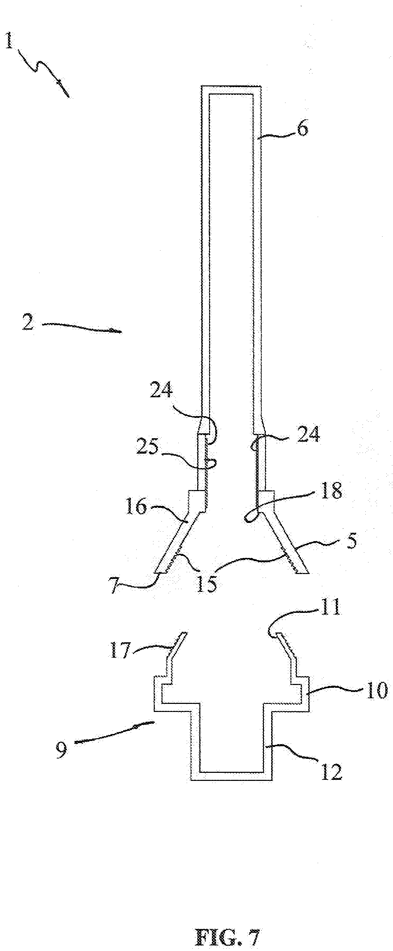

**Fig. 7** partially shows a second embodiment of the assembly **1** which differs from that described above essentially by the fact that the coupling means are of the screw and nut type and are defined by opposite threads of the frustoconical proximal portion **5** of the cap **2** and of the outer surface **17** of the closure cap **9.**

In both of the illustrated embodiments it is possible to observe that the proximal end portion **5** comprises an inner annular surface **18** adapted to define a front abutment for the hub **8,** so as to limit the axial displacement of the tubular protective cap **2** toward the inside.

In the first embodiment, the proximal end portion **5** has an inner section defining a shaped seat **19** for housing in a snugly fit manner a counter-shaped portion of the hub **8.**

In particular, the hub **8** has a proximal cylindrical section **8'** and a tapered distal portion **8"** with a curvilinear profile designed to be inserted in a snugly fit manner in the housing seat **19** and which means for fastening into rotation to the protective element **2** are also associated to.

In a known manner, the hub **8** has an inner axial passage **20** for inserting and locking the needle **3** and is internally threaded to mate by screwing on the syringe or similar injection apparatus.

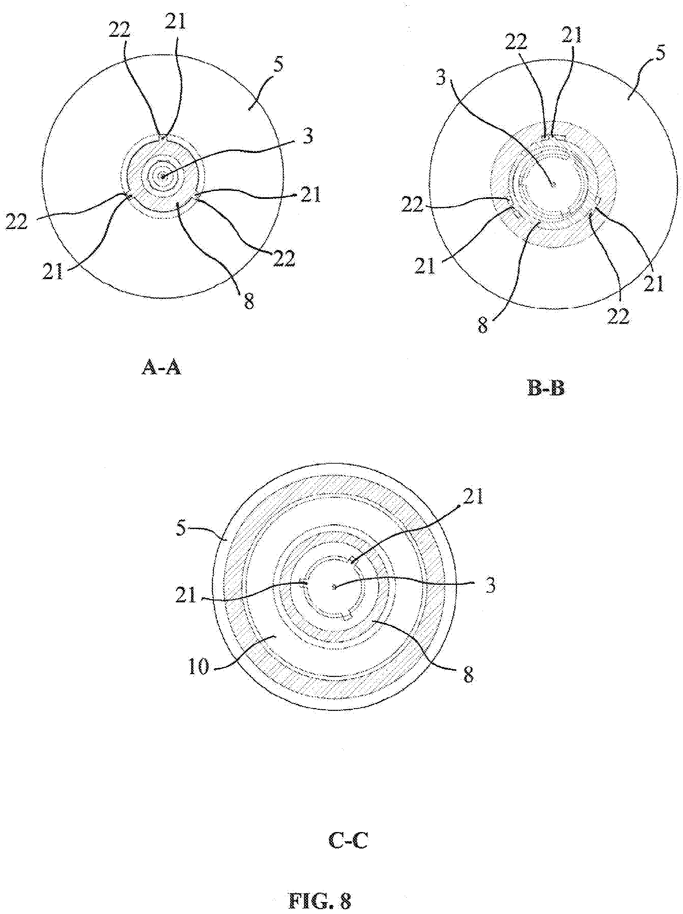

The rotation fastening means comprise axial projections **21** which extend along the outer surface of the tapered portion **8"** of the hub **8** to be inserted into corresponding axial grooves **22** made in the peripheral wall **16** of the proximal end portion **5** of the protective element **2,** as more clearly visible from the cross sections of **Fig. 8****.**

The projections **21** and the grooves **22** operate both as rotation fastening means to facilitate removal of the hub **8** and of the needle **3** from the syringe unitarily to the cap **2** as a result of a rotation movement imparted by the operator around a common central rotation axis, corresponding with the longitudinal axis **L,** and as guide means to facilitate the correct insertion of the needle **3** inside the cap **2.**

In the embodiment of **Fig. 7** two or more axial fins **24** are provided and project from an inner cylindrical surface **25** of the tubular protection cap **2** to engage the projections **21** of the hub **8** upon the reciprocal rotation.

Not least, according to a not shown variant axial fastening means may be provided for fastening the cap **9** and the hub **8** and which may comprise one or more radial fins extending from the lower edge **23** of the hub **8** to fit in the second peripheral grooves of the closure cap **9** following upon their relative rotation about the longitudinal axis **L.**

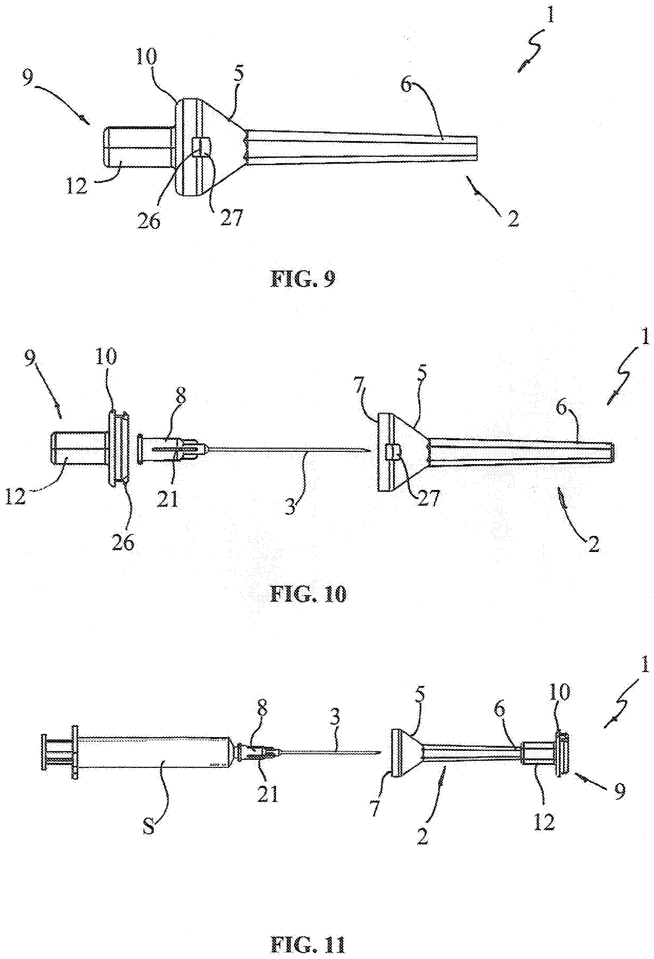

**Figs. 9** and **10** show a third embodiment of the assembly **1** that differs from the first primarily for the fact that the coupling means between the discoidal element **10** and the proximal end portion **5** of the cap **2** are of the irreversible type, i.e, do not allow the reopening of the cap **2** once it has been locked on the closure cap **9.**

The irreversibility is obtained by means of a pair of radial teeth **26** of the discoidal element **10** adapted to snap fit into respective peripheral seats **27** of the proximal end portion **5** of the cap **2** upon the relative rotation between the respective parts.

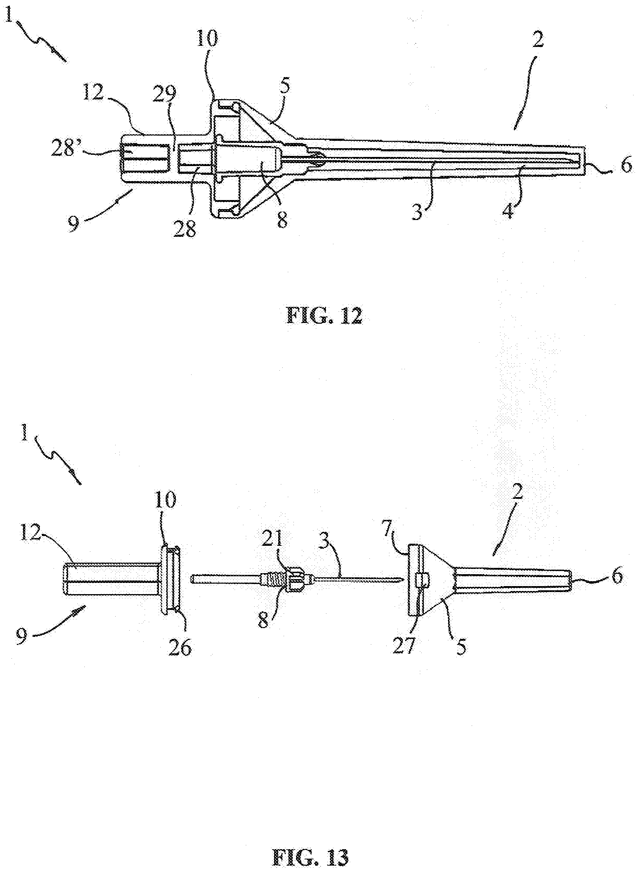

A further difference, visible in **Fig. 12****,** is represented by the fact that the tubular central body **12** of the closure cap **9** comprises a pair of coaxial cavities **28, 28'** separated by a radial baffle **29** and each having an inner shape complementary to the outer shape of the distal end portion **6** for the selective fitting thereof.

In this way the assembly **1** will allow both a direct capping as shown in **Fig. 2****,** wherein the operator will handle the cap **2** with one hand through the closure cap **9** while with the other hand will grasp the syringe, that an indirect capping, shown in **Fig. 11****,** wherein the cap **9** may be placed on a horizontal plane to lie on the discoidal element **10** and hold the cap **2** in a vertical position.

In this way, the health care worker may insert the needle **3** into cap **2** without having to get a finger in front of the needle **3,** further increasing the safety of the assembly **1.**

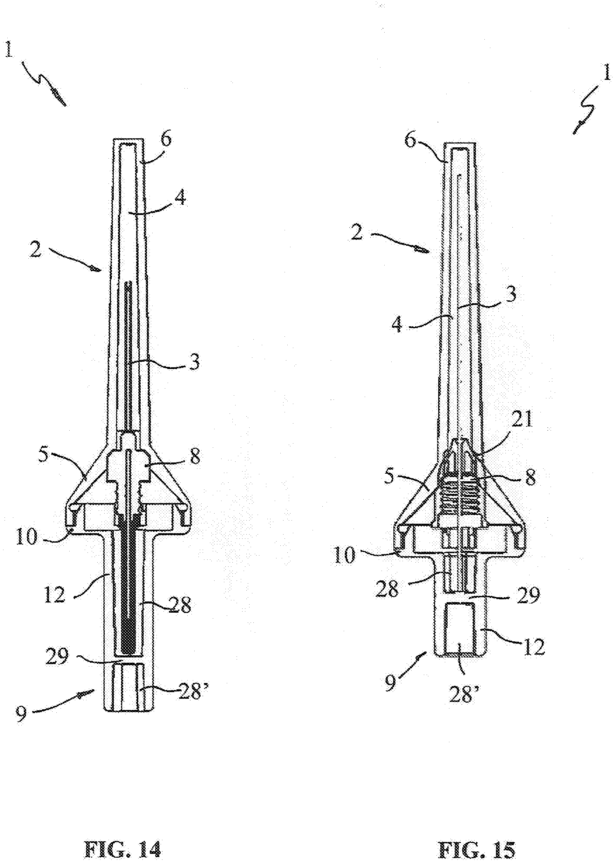

**Figs. 13** and **14** show a fourth embodiment of the assembly **1** that differs from the previous by the fact that it houses a needle **3** known as vacutainer, used for venepuncture.

Finally, **Fig. 15** shows a fifth embodiment that differs for the presence of a dental needle **3.**

However, it is understood that the assembly **1** may be used in association with any type of needle, without particular theoretical limitations.

In addition, variants of all the shown embodiments are also possible without requiring the presence of the rotation fastening means between the hub **8** of the needle **3** and the cap **2.**

From above it appears evident that the invention achieves the intended objects and in particular that of making available a safe needle assembly for protection against needles pricks that is applicable in a simple and safe manner to a used needle to allow its recapping and dispose of in a safe manner and in conformity with the current regulations.

The assembly according to the invention is susceptible of numerous modifications and variations all falling within the inventive concept expressed in the accompanying claims. All the details may furthermore be replaced with other technically equivalent elements, and the materials may be different according to requirements, without departing from the scope of protection of the present invention.

In particular, the non-metallic parts of the assembly, i.e. those different from the needle, may be made of a polymer material of adequate strength and suitable for disposal in accordance with local regulations.

Even if the assembly has been disclosed with particular reference to the attached figures, the reference numbers used in the description and in the claims are used to improve the intelligence of the invention and do not constitute any limitation to the claimed scope of protection.

## Claims

1. A safe needle assembly for protection against needle pricks, comprising:
- a needle (**3**) having one end (**3'**) adapted to be coupled to a syringe or the like containing a fluid to be supplied and the opposite end (**3"**) adapted to supply the fluid to be injected;
- a hub (**8**) for removably anchoring said needle (**3**) to the syringe or the like, said hub (**8**) having an inner axial passage (**20**) for inserting and locking said needle (**3**) and an outer surface with a proximal cylindrical section (**8'**) and a tapered distal section (**8"**);
- an internally hollow protective cap (**2**) with an open proximal end portion (**5**) allowing the insertion of at least said needle (**3**) and a distal end portion (**6**), said proximal end portion (**5**) having frustoconical shape tapered toward said distal portion (**6**) with an enlarged inlet (**7**) to facilitate the insertion of said hub (**8**) in to said protective cap (**2**) and to guide the insertion of said needle (**3**) into said distal end portion (**6**);
- a closure cap (**9**) having a discoidal element (**10**) adapted to be selectively coupled to one of said proximal tapered end portion (**5**) for closing said enlarged inlet (**7**) and said distal end portion (**6**) for the safe handling of said protection cap (**2**);
wherein said closure cap (**9**) has a central through hole (**11**) for positioning said discoidal element (**10**) on said distal end portion (**6**) when not coupled to said proximal end portion (**5**);
wherein said protective cap (**2**) and said hub (**8**) respectively have first and second rotational fastening means adapted to make said needle (**3**) integral in rotation to said protective cap (**2**) and wherein said discoidal element (10) and said proximal end portion (5) of said cap (2) have respective mutual coupling means (13, 14, 15, 16) complementary shaped with each other, and **characterized in that** said coupling means (**13, 14, 15, 16**) are selected between screw and nut means, snap fit means, bayonet means, male and female means and the like, said coupling means being of the irreversible type.

2. Safe needle assembly as claimed in claim 1, **characterized in that** said proximal end portion (**5**) of said protective cap (**2**) has an inner peripheral surface (**16**) provided with said first rotational fastening means (**22**).

3. Safe needle assembly as claimed in claim 2, **characterized in that** said first rotational fastening means comprise one or more axial grooves (**22**) formed in said inner peripheral surface (**16**) of said proximal end portion (**5**) of said protective cap (**2**) and adapted to house said second rotational fastening means.

4. Safe needle assembly as claimed in claim 3, **characterized in that** said second rotational fastening means comprise one or more axial projections (**21**) that extend along the outer surface of said hub (**8**) for engaging in a snugly fit manner corresponding axial grooves (**22**) of said first rotational fastening means.

5. Safe needle assembly as claimed in any preceding claim, **characterized in that** said closure cap (**9**) comprises a central tubular body (**12**) that extends from said discoidal element (**10**) coaxially to said central through hole (**11**) and having an inner shape complementary to the outer shape of said distal end portion (6) for housing to measure thereof.

6. Safe needle assembly as claimed in claim 5, **characterized in that** said central tubular body (**12**) of said closure cap (**9**) comprises a pair of coaxial cavities (**28, 28'**) separated by a radial baffle (**29**) and each having an inner shape complementary to the outer shape of said distal end portion (**6**) for selectively housing thereof in a snugly fit manner.

7. Safe needle assembly as claimed in any preceding claim, **characterized in that** said discoidal element (**10**) has an outer maximum diameter substantially equal to the outer diameter of said enlarged inlet (**7**).

8. Safe needle assembly as claimed in any preceeding claim, **characterized in that** said proximal end portion (**5**) comprises one or more radial fins (**13**) extending internally from said inlet (**7**) to be inserted into correspondent first perimeter grooves (**14**) of said discoidal element (**10**) upon the relative rotation between said closure cap (**9**) and said protective cap (**2**) for their mutual firm and removable coupling.

9. Safe needle assembly as claimed in any preceding claim, **characterized in that** said proximal end portion (**5**) of said protective cap (**2**) comprises an inner annular surface (**18**) adapted to define a front abutment for said hub (**8**).

10. Safe needle assembly as claimed in any preceding claim, **characterized in that** said hub (**8**) comprises one or more radial fins adapted to be inserted by rotation in corresponding second perimeter grooves of said closure cap (**9**) for axially fastening said needle (**3**) for the mutual axial fastening thereof.

## Patentansprüche

1. Sichere Nadelanordnung zum Schutz gegen Nadelstiche, umfassend:
- eine Nadel (3) mit einem Ende (3'), das mit einer Spritze oder dergleichen gekoppelt werden kann, die ein zuzuführendes Fluid enthält, und dem gegenüberliegenden Ende (3"), das das zu injizierende Fluid zuführt;
- eine Nabe (8) zum lösbaren Verankern der Nadel (3) an der Spritze oder dergleichen, wobei die Nabe (8) einen inneren axialen Durchgang (20) zum Einführen und Verriegeln der Nadel (3) und eine äußere Oberfläche mit einer proximalen Oberfläche aufweist zylindrischer Abschnitt (8') und ein sich verjüngender distaler Abschnitt (8");
- eine innen hohle Schutzkappe (2) mit einem offenen proximalen Endabschnitt (5), der das Einführen von mindestens der Nadel (3) und einem distalen Endabschnitt (6) ermöglicht, wobei der proximale Endabschnitt (5) eine kegelstumpfförmige Form aufweist, die sich in Richtung verjüngt wobei der distale Abschnitt (6) einen vergrößerten Einlass (7) aufweist, um das Einführen der Nabe (8) in die Schutzkappe (2) zu erleichtern und das Einführen der Nadel (3) in den distalen Endabschnitt (6) zu führen.;
- eine Verschlusskappe (9) mit einem scheibenförmigen Element (10), das zur sicheren Handhabung wahlweise mit dem proximalen konischen Endabschnitt (5) zum Verschließen des vergrößerten Einlasses (7) und des distalen Endabschnitts (6) gekoppelt werden kann der Schutzkappe (2); wobei die Verschlusskappe (9) ein zentrales Durchgangsloch (11) zum Positionieren des scheibenförmigen Elements (10) auf dem distalen Endabschnitt (6) aufweist, wenn es nicht mit dem proximalen Endabschnitt (5) gekoppelt ist;
wobei die Schutzkappe (2) und die Nabe (8) jeweils ein erstes und ein zweites Drehbefestigungsmittel aufweisen, um die Nadel (3) einstückig mit der Schutzkappe (2) zu drehen; wobei das scheibenförmige Element (10) und der proximale Endabschnitt (5) der Kappe (2) jeweilige gegenseitige Kopplungsmittel (13, 14, 15, 16) aufweisen, die komplementär zueinander geformt sind und
**dadurch gekennzeichnet, dass** die Kopplungsmittel (13, 14, 15, 16) ausgewählt sind aus Schraub- und Mutternmitteln, Schnappmitteln, Bajonettmitteln, männlichen und weiblichen Mitteln und dergleichen, wobei die Kopplungsmittel vom irreversiblen Typ sind.

2. Sichere Nadelanordnung nach Anspruch 1, **dadurch gekennzeichnet, dass** der proximale Endabschnitt (5) der Schutzkappe (2) eine Innenumfangsfläche (16) aufweist, die mit dem ersten Drehbefestigungsmittel (22) versehen ist.

3. Sichere Nadelanordnung nach Anspruch 2, **dadurch gekennzeichnet, dass** die ersten Drehbefestigungsmittel eine oder mehrere axiale Nuten (22) aufweisen, die in der Innenumfangsfläche (16) des proximalen Endabschnitts (5) der Schutzkappe (16) ausgebildet sind. 2) und angepasst, um das zweite Drehbefestigungsmittel aufzunehmen.

4. Sichere Nadelanordnung nach Anspruch 3, **dadurch gekennzeichnet, dass** die zweiten Drehbefestigungsmittel einen oder mehrere axiale Vorsprünge (21) umfassen, die sich entlang der Außenfläche der Nabe (8) erstrecken, um in passgenaue Weise in entsprechende axiale Nuten einzugreifen (22) des ersten Drehbefestigungsmittels.

5. Sichere Nadelanordnung nach einem der vorhergehenden Ansprüche, **dadurch gekennzeichnet, dass** die Verschlusskappe (9) einen zentralen röhrenförmigen Körper (12) umfasst, der sich von dem scheibenförmigen Element (10) koaxial zu dem zentralen Durchgangsloch (11) erstreckt und eine innere Form, die zu der äußeren Form des distalen Endabschnitts (6) komplementär ist, um ein Gehäuse zu schaffen, um dieses zu messen.

6. Sichere Nadelanordnung nach Anspruch 5, **dadurch gekennzeichnet, dass** der zentrale röhrenförmige Körper (12) der Verschlusskappe (9) ein Paar koaxialer Hohlräume (28, 28 ') umfasst, die durch eine radiale Trennwand (29) voneinander getrennt sind mit einer inneren Form, die zu der äußeren Form des distalen Endabschnitts (6) komplementär ist, um diesen selektiv in einer passgenauen Weise aufzunehmen.

7. Sichere Nadelanordnung nach einem der vorhergehenden Ansprüche, **dadurch gekennzeichnet, dass** das scheibenförmige Element (10) einen maximalen Außendurchmesser aufweist, der im Wesentlichen gleich dem Außendurchmesser des vergrößerten Einlasses (7) ist.

8. Sichere Nadelanordnung nach einem der vorhergehenden Ansprüche, **dadurch gekennzeichnet, dass** der proximale Endabschnitt (5) eine oder mehrere radiale Rippen (13) umfasst, die sich von dem Einlass (7) nach innen erstrecken, um in entsprechende erste Umfangsnuten (14) eingeführt zu werden, des scheibenförmigen Elements (10) bei der Relativdrehung zwischen der Verschlusskappe (9) und der Schutzkappe (2) für ihre gegenseitige feste und entfernbare Kopplung.

9. Sichere Nadelanordnung nach einem der vorhergehenden Ansprüche, **dadurch gekennzeichnet, dass** der proximale Endabschnitt (5) der Schutzkappe (2) eine innere ringförmige Oberfläche (18) aufweist, die angepasst ist, um ein vorderes Widerlager für die Nabe (8) zu definieren.

10. Sichere Nadelanordnung nach einem der vorhergehenden Ansprüche, **dadurch gekennzeichnet, dass** die Nabe (8) eine oder mehrere radiale Rippen umfasst, die durch Drehen in entsprechende zweite Umfangsnuten der Verschlusskappe (9) eingeführt werden können, um die Nadel (9) axial zu befestigen. 3) zu deren gegenseitigen axialen Befestigung.

## Revendications

1. Assemblage d'aiguille sûr pour la protection contre les piqûres d'aiguille, comprenant
- une aiguille (3) ayant une extrémité (3') adaptée pour être couplée à une seringue ou analogue contenant un fluide à alimenter et l'extrémité opposée (3) adaptée à alimenter le fluide à injecter;
- un moyeu (8) pour ancrer de manière amovible ladite aiguille (3) à la seringue ou analogue, ledit moyeu (8) comportant un passage axial interne (20) pour insérer et verrouiller ladite aiguille (3) et une surface extérieure avec une surface proximale une section cylindrique (8') et une section distale effilée (8");
- un capuchon protecteur creux intérieurement (2) avec une partie d'extrémité proximale ouverte (5) permettant l'insertion d'au moins ladite aiguille (3) et une partie d'extrémité distale (6), ladite partie d'extrémité proximale (5) ayant une forme tronconique effilée vers ladite partie distale (6) avec une entrée élargie (7) pour faciliter l'insertion dudit moyeu (8) dans ledit capuchon protecteur (2) et pour guider l'insertion de ladite aiguille (3) dans ladite partie d'extrémité distale (6) ;
- un capuchon de fermeture (9) ayant un élément discoïdal (10) adapté pour être couplé sélectivement à l'une de ladite partie d'extrémité effilée proximale (5) pour fermer ladite entrée agrandie (7) et ladite partie d'extrémité distale (6) pour une manipulation en toute sécurité dudit capuchon de protection (2);
dans lequel ledit capuchon de fermeture (9) a un trou traversant central (11) pour positionner ledit élément discoïdal (10) sur ladite partie d'extrémité distale (6) lorsqu'il n'est pas couplé à ladite partie d'extrémité proximale (5);
dans lequel ledit capuchon protecteur (2) et ledit moyeu (8) ont respectivement des premier et second moyens de fixation rotatifs adaptés pour rendre ladite aiguille (3) solidaire en rotation dudit capuchon protecteur (2);
dans lequel ledit élément discoïdal (10) et ladite partie d'extrémité proximale (5) dudit capuchon (2) ont des moyens de couplage mutuels respectifs (13, 14, 15, 16) de forme complémentaire, et
**caractérisé en ce que** lesdits moyens de couplage (13, 14, 15, 16) sont choisis entre des moyens vis et écrou, des moyens à encliquetage, des moyens à baïonnette, des moyens mâle et femelle et similaires, lesdits moyens de couplage étant du type irréversible.

2. Assemblage d'aiguille sécurisée selon la revendication 1, **caractérisé en ce que** ladite partie d'extrémité proximale (5) dudit capuchon de protection (2) présente une surface périphérique interne (16) munie dudit premier moyen de fixation rotatif (22).

3. Assemblage d'aiguilles sûres selon la revendication 2, **caractérisé en ce que** lesdits premiers moyens de fixation en rotation comprennent une ou plusieurs rainures axiales (22) formées dans ladite surface périphérique intérieure (16) de ladite partie d'extrémité proximale (5) dudit capuchon protecteur (2) et adapté pour loger ledit second moyen de fixation par rotation.

4. Assemblage d'aiguilles sûres selon la revendication 3, **caractérisé en ce que** lesdits deuxièmes moyens de fixation en rotation comprennent une ou plusieurs saillies axiales (21) qui s'étendent le long de la surface extérieure dudit moyeu (8) pour s'engager de manière parfaitement ajustée dans les rainures axiales correspondantes. (22) desdits premiers moyens de fixation par rotation.

5. Assemblage d'aiguilles sûres selon l'une quelconque des revendications précédentes, **caractérisé en ce que** ledit capuchon de fermeture (9) comprend un corps tubulaire central (12) qui s'étend coaxialement audit élément discoïdal (10) audit trou traversant central (11) et forme intérieure complémentaire à la forme extérieure de ladite partie d'extrémité distale (6) pour logement afin de mesurer celle-ci.

6. Assemblage d'aiguilles sûres selon la revendication 5, **caractérisé en ce que** ledit corps tubulaire central (12) dudit capuchon de fermeture (9) comprend une paire de cavités coaxiales (28, 28 ') séparées par un déflecteur radial (29) et chaque ayant une forme interne complémentaire à la forme externe de ladite partie d'extrémité distale (6) pour loger de manière sélective celle-ci d'une manière parfaitement ajustée.

7. Assemblage d'aiguilles sûres selon l'une quelconque des revendications précédentes, **caractérisé en ce que** ledit élément discoïdal (10) a un diamètre extérieur maximal sensiblement égal au diamètre extérieur de ladite entrée élargie (7).

8. Assemblage d'aiguilles sûres selon l'une quelconque des revendications précédentes, **caractérisé en ce que** ladite partie d'extrémité proximale (5) comprend une ou plusieurs ailettes radiales (13) s'étendant intérieurement depuis ladite entrée (7) pour être insérées dans les premières rainures de périmètre correspondantes (14), dudit élément discoïdal (10) lors de la rotation relative entre ledit capuchon de fermeture (9) et ledit capuchon de protection (2) pour leur couplage ferme et amovible mutuel.

9. Assemblage d'aiguilles sûres selon l'une quelconque des revendications précédentes, **caractérisé en ce que** ladite partie d'extrémité proximale (5) dudit capuchon protecteur (2) comprend une surface annulaire interne (18) adaptée pour définir une butée avant pour ledit moyeu (8).

10. Assemblage d'aiguille sécurisée selon l'une quelconque des revendications précédentes, **caractérisé en ce que** ledit moyeu (8) comprend une ou plusieurs ailettes radiales adaptées pour être insérées par rotation dans des secondes rainures périmétriques correspondantes dudit capuchon de fermeture (9) pour fixer axialement ladite aiguille (3) pour leur fixation axiale mutuelle.
